(19) Europäisches Patentamt
European Patent Office
Office européen des brevets

(11) EP 1 291 342 A1

(12) EUROPEAN PATENT APPLICATION

(43) Date of publication:
**12.03.2003 Bulletin 2003/11**

(51) Int Cl.[7]: **C07D 213/65**, A23L 1/22

(21) Application number: **01121349.3**

(22) Date of filing: **06.09.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **SOCIETE DES PRODUITS NESTLE S.A.**
**1800 Vevey (CH)**

(72) Inventors:
- **Hofmann, Thomas**
**86375 Neufahrn (DE)**
- **Ottinger, Harald**
**80805 München (DE)**
- **Frank, Oliver**
**85375 Neufahrn (DE)**
- **Soldo, Tomislav**
**87700 Memmingen (DE)**
- **Blank, Imre**
**1073 Savigny (CH)**
- **Villard, Renaud**
**1084 Carrouge (CH)**
- **Robert, Fabien**
**01220 Divonne les Bains (FR)**

(74) Representative: **Thomas, Alain et al**
**55, avenue Nestlé**
**1800 Vevey (CH)**

(54) **Pyridinium-betain compounds as taste enhancer**

(57) The present invention concerns Pyridinium-Betain compounds of the following general formula (**A**)

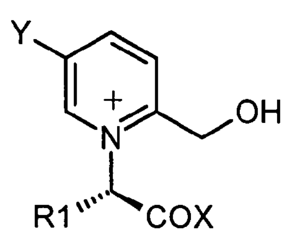

**(A)**

wherein R1 is taken from the group consisting of a rest of primary L-amino acid,
X is taken from the group consisting of OH and $O^-$
Y is taken from the group consisting of OH and $O^-$
and wherein the counter-ion is taken from the group consisting of sodium, potassium, ammonium, calcium, magnesium, chloride, nitrate, carbonate, sulfate, phosphate, and thelike.

EP 1 291 342 A1

## Description

The present invention concerns Pyridinium-Betain compounds and their use as taste active compounds or taste enhancing compounds.

The non-enzymatic reaction between reducing carbohydrates and amino acids, named as the Maillard reaction, is known to generate aroma, taste and browning compounds contributing to the sensory quality of thermally processed foods, such as processed meats, cereals, malt, coffee or cocoa.

Compared to aroma-active volatiles, the information available on culinary taste compounds generated during thermal food processing is as yet very fragmentary. The structures and sensory qualities of Maillard reaction products exhibiting bitter, salty, sweet or umami taste, respectively, as well as components eliciting certain additional effects on the tongue such as, e.g. heating, cooling, astringency etc., are as yet not known. Recently, we developed a novel bioassay, which is based on the determination of the taste threshold of reaction products in serial dilutions of HPLC fractions (Frank, O., Ottinger, H., Hofmann T. *J. Agric. Food Chem.* **2001,** *49*, 231-238; Ottinger, H., Bareth, A., Hofmann, T. *J. Agric. Food Chem.* **2001,** 49, 1336-1344), to select the most intense taste compounds in a thermally treated solution of xylose and primary amino acids exhibiting intense bitter taste. By application of this so called Taste Dilution Analysis (TDA) as a powerful screening procedure for the detection of as yet unknown taste-active compounds in combination with LC/MS as well as 1D-and 2D-NMR experiments the previously unknown 3-(2-furyl)-8-[(2-furyl)methyl]-4-hydroxymethyl-1-oxo-1*H*,4*H*-quinolizinium-7-olate could be identified as the key tastant in the reaction mixture. This novel compound, which was named Quinizolate, exhibited an intense bitter taste at an extraordinarily low detection threshold of 0.00025 mmol/kg water.

By application of this technique on roasted glucose/proline mixtures, we were able to successfully detect, besides various bitter and astringent tasting compounds, also two products exhibiting an intense cooling effect on the tongue, which were identified as 5-methyl-2-(1-pyrrolidinyl)-2-cyclopenten-1-one and 3-methyl-2-(1-pyrrolidinyl)-2-cyclo-penten-1-one (Ottinger, H., Bareth, A., Hofmann, T. *J. Agric. Food Chem.* **2001,** 49, 1336-1344; European Patent Application No. 00110886.9 filed May 23, 2000). These are the first compounds reported to possess strong cooling activity on the tongue without exhibiting a prominent minty odor as in the case of, e.g., menthol. The knowledge of the structures and sensory properties of such "tongue-affecting" compounds would offer new possibilities to evoke certain taste effects during consumption of food compositions.

The aim of the present invention is to identify compounds having sensory properties, for example to impart a taste-active or a taste enhancement quality.

The present invention concerns new compounds called Pyridinium-Betain compounds of the general formula **(A)** :

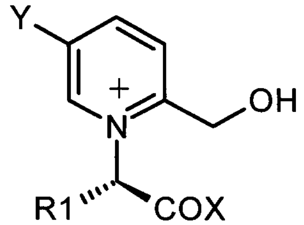

**(A)**

wherein

R1 is taken from the group consisting of a rest of primary L-amino acids,
X is taken from the group consisting of OH and $O^-$
Y is taken from the group consisting of OH and $O^-$

and wherein the counter-ion is taken from the group consisting of sodium, potassium, ammonium, calcium, magnesium, chloride, nitrate, carbonate, sulfate, phosphate, and thelike.

The compounds of the general formula **(A)** have the (*S*)-stereochemistry according to the Cahn-Ingold-Prelog nomenclature.

R1 can be taken from the range of primary L-amino acid, for example from alanine, valine, leucine, isoleucine, phenylalanine, tryptophan, methionine, serine, threonine, cysteine, tyrosine, asparagine, glutamine, aspartic acid, glutamic acid, lysine, 5-hydroxylysine, ornithine, histidine and arginine. The list is not exhaustive and can be completed

by any other primary amino acids. Preferably, R1 is the rest of alanine, phenylalanine, and glutamic acid. The following specifications give more details on several compounds according to the invention.

The compounds of the general formula **(A)** have zwitterionic character in a broad pH range. As shown below, the zwitterionic structure **(A2)** dominate under neutral conditions with the negative charge primarily located at the carbocylic group (X in **(A)**). Under basic conditions represented by the structure **(A3),** the negative charge may be located at the carboxylic group of the amino acid moiety (group X in **(A)**) and at the hydroxyl group directly attached to the pyridinium ring (group Y in **(A)**). Under strongly acidic conditions, both the carboxylic and hydroxyl groups are protonated, as shown in the structure **(A1),** i.e. the groups X and Y correspond to OH. Depending on the pH of an aqueous solution containing the compounds of the general formula **(A),** the structures **(A1), (A2)** and **(A3)** may exist in equilibrium.

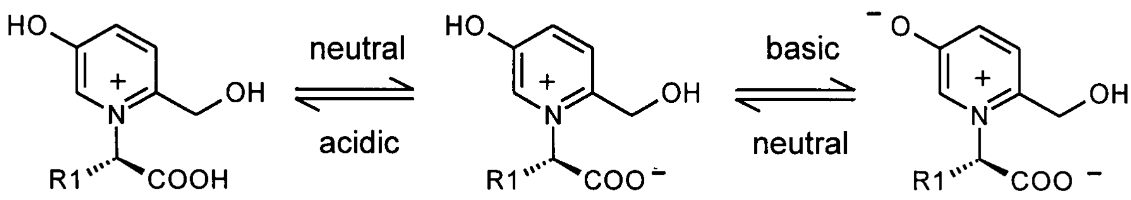


**(A1)** **(A2)** **(A3)**

The rest R1 of the compounds of the general formula **(A)** may also be included in the charge distribution. For example, the γ-carboxylic group of L-glutamic acid may be negatively charged depending on the pH, as shown below.

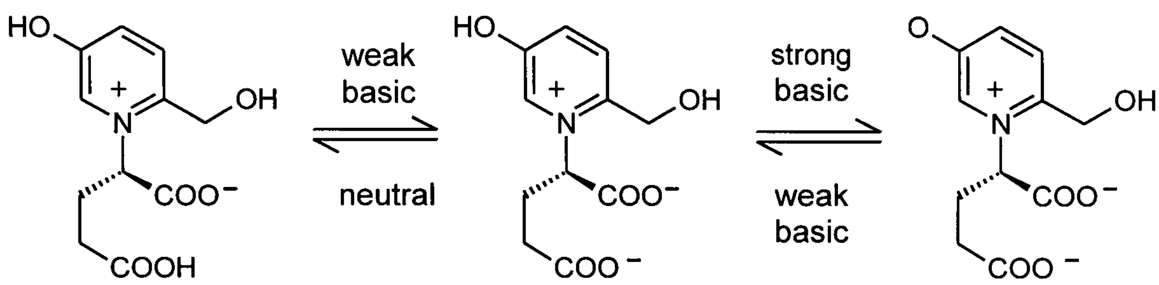


**(A4)** **(A5)** **(A6)**

Similarly, the ε-amino group of L-lysine may be positively charged depending on the pH.

The above mentioned compounds are reaction products from a reducing sugar with a primary L-amino acid or a peptide.

Considering a more specific reaction and by application of the taste dilution analysis on HPLC fractions obtained from a thermally treated solution of glucose and L-alanine, a compound could be detected, which imparts an intense sweet taste upon sensory evaluation. This compound was found to be formed in higher concentrations when the hexose degradation product 5-hydroxymethyl-2-furanaldehyde (HMF) was reacted with L-alanine. After isolation by column chromatography on RP-18 material the taste compound could be obtained as pale-yellow crystals with high purity. LC/MS and 1D- and 2D-NMR spectroscopy led to the unequivocal identification of the compound as (*S*)-N-(1-carboxyethyl)-6-hydroxymethylpyridinium-3-olate.

The present invention concerns further the new compounds of general formula **(A),** wherein X of COX corresponds to a further amino acid or oligopeptides attached *via* peptidic bonds. In these compounds, R1 may be a proton (R1= H). The amino acids concerned here are the same as the above mentioned primary L-amino acids and, in addition,

glycine and secondary L-amino acids such as for example L-proline and L-4-hydroxyproline. The oligopeptides concerned may be comprised of primary and secondary L-amino acids including glycine. Again, the charge distribution in the molecule of the general formula **(A)** depend on the pH and may also include the rest of the amino acid or peptide.

For example, the negative charge can be located at both the carboxylic group of the peptide and the hydroxyl group directly attached to the pyridinium ring.

The present invention concerns further the use of the above mentioned Pyridinium-Betain compounds, wherein compound **(A)** is added to a food composition for imparting to said food a sweetness, a bitterness, a salty taste or an umami taste. Some of the compounds of the general formula **(A)** have intense taste properties from sweetness to bitterness via salty and umami tasting. So, these compounds can be added to flavor some food compositions. The possible food compositions are taken from the group consisting of chocolate, ice-cream, beverages, sugar confectionery, culinary products, and petfood. Under culinary products, we understand any dehydrated, shelf stable, frozen or chilled product, like soups, mayonnaise, gravies, sauces, and the like.

The invention concerns finally the use of the new Pyridinium-Betain compounds **(A),** wherein compound **(A)** is added to a food composition to enhance the sweetness, the bitterness, the salty taste or the umami taste of a compound having said functionality. In the use of enhancer, two ways are possible. Either, the compound **(A)** has the same functionality as the food composition where it is added or the compound **(A)** has another functionality of the food composition where it is added. For example, it is possible to use a compound **(A)** having a sweet taste to enhance the sweetness of a food composition containing for example glucose. However, it is also possible to use the same sweet tasting compound **(A)** to enhance the umami taste of a food composition containing for example monosodium glutamate (MSG).

In the case of both uses, the amount of the compound **(A)** is comprised between 0.01 and 3000 mg/kg of the whole composition.

The present invention concerns finally a process for the preparation of the compounds **(A),** wherein said compound is obtained by synthesis using 5-(hydroxymethyl)-2-furanaldehyde and the corresponding L-amino acid or peptides. Another way of proceeding is to use HMF producing precursors, such mono- and polysaccharides, and the corresponding L-amino acid or peptides.

The following examples illustrate the invention in more details.

**Syntheses of (S)-N-(1-carboxyethyl)-6-hydroxymethylpyridinium-3-olate**

**[(*S*)-Alapyridaine] ( R1 is the rest of L-alanine )**

***1. First way of preparation: From glucose and L-alanine in water.***

A solution of D-glucose (200 mmol) and L-alanine (400 mmol) in phosphate buffer (400 mL; 1.0 mol/L, pH 5.0) was heated under reflux for 30 min. After cooling to room temperature, the aqueous solution was extracted with ethyl acetate (3 × 100 mL), the aqueous layer was freeze-dried, the residue obtained was taken up in water (20 mL) and then separated by column chromatography (4 x 20 cm) using RP-18 material (LiChroprep 25 - 40 μm, Merck, Darmstadt, Germany) as the stationary phase and a mixture (97/3, v/v) of ammonium formate (10 mmol/l, pH 8.2) and methanol as the mobile phase. Monitoring the effluent at 252 nm, the target compound was eluted between 140 and 190 mL as confirmed by RP-HPLC/taste testing. After freeze-drying, (*S*)-N-(1-carboxyethyl)-6-hydroxymethyl-pyridinium-3-olate (1.2 mmol, 0.6 % yield) was obtained with a purity of more than 99%.

***2. Second way of preparation:*** **From** ***5-(hydroxymethyl)-2 furanaldehyd (HMF) and L-alanine (see below).***

A solution of 5-(hydroxymethyl)-2-furanaldehyde (25 mmol) and L-alanine (35 mmol) in water/ethanol (1/1, v/v; 60 mL) was adjusted to pH 9.4 with conc. sodium hydroxide solution and then refluxed for 3 days. After cooling to room temperature the reaction mixture was freed from solvent *in vacuo* (45 mbar), taken up in water (20 mL), and the aqueous solution was extracted with ethyl acetate (2 x 10 mL). Aliquots (5 mL) of the aqueous phase were then applied onto the top of a water-cooled glass column (4 x 20 cm) filled with a slurry of RP-18 material (LiChroprep 25 - 40 μm, Merck) in a mixture (97/3, v/v) of ammonium formate (10 mmol/l, pH 8.2) and methanol. Using the same solvent mixture as the mobile phase and monitoring the effluent at 252 nm, the target compound was eluted between 140 and 190 mL as confirmed by RP-HPLC/taste testing. After freeze-drying, (*S*)-N-(1-carboxyethyl)-6-hydroxymethyl-pyridinium-3-olate **(A7)** was obtained (12.8 mmol, 51 % yield) with a purity of more than 99%.

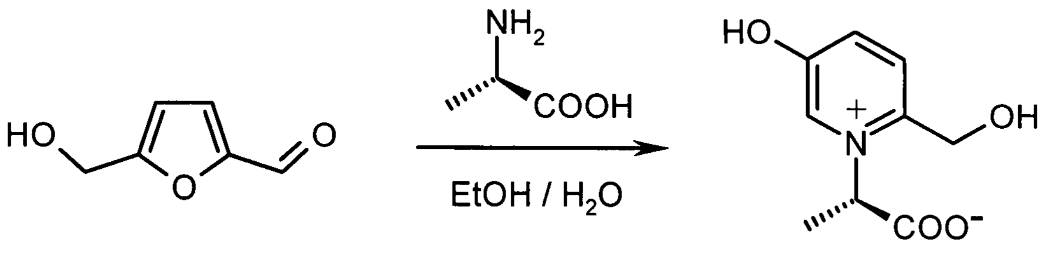


**(A7)**

**Spectroscopic data of (*S*)-Alapyridaine:**

**LC/MS** (ESI): 198 (100, $[M+1]^+$), 220 (57, $[M+Na]^+$), 395 (19, $[2 M+1]^+$), 417 (29, $[2 M+Na]^+$).
**LC/MS** ($ESI^-$) : 197 (100, $[M]^+$).
**UV/VIS** (ammonium formate; 10 mmol/L; pH 8.2): $\lambda_{max}$ = 251, 328 nm.
**$^1$H NMR** (360 MHz; DMSO-$d_6$, GRASP-COSY): δ 1.81 (d, 3H, J=7.08 Hz, $CH_3$), 4.69 (d, J=13.27, 1H, $CH_aH$), 4.78 (d, J=13.27, 1H, $CHH_b$), 5.23 (q, 1H, J=7.08, CH), 7.06 (dd, J=2.65, 8.85 Hz, 1H, CH), 7.45 (d, J=8.85 Hz, 1H, CH), 7.50 (d, J=2.65 Hz, 1H, CH), 8.78 (bs, 1H, COOH).
**$^{13}$C NMR** (360 MHz; DMSO-$d_6$, DEPT, HSQC, HMBC): δ 19.0 [$CH_3$], 59.1 [$CH_2$], 64.1 [CH], 127.6 [CH], 131.3 [CH], 131.8 [C], 132.3 [CH], 166.0 [C], 167.7 [COOH].

**Syntheses of (S)-N-(1-carboxy-2-phenylethyl)-6-hydroxymethyl-pyridinium-3-olate [(*S*)-Phepyridaine] ( R1 is the rest of L-Phenylalanine )**

***From 5-(hydroxymethyl)-2-furanaldehyde (HMF) and L-phenylalanine.***

A solution of 5-(hydroxymethyl)-2-furanaldehyde (25 mmol) and L-phenylalanine (35 mmol) in water/ethanol (1/1, v/v, 100 ml) was adjusted to pH 9.4 with conc. potassium hydroxide solution and then refluxed for 2 days. After cooling to room temperature the reaction mixture was freed from solvent in vacuo (45 mbar), taken up in water (20 mL), and the aqueous phase was extracted with ethyl acetate (2 x 10 mL). Aliquots (10 mL) of the aqueous phase were applied onto the top of a water-cooled glass column (4 x 30 cm) filled with a slurry of RP-18 material (LiChroprep 25 - 40 μm, Merck) in a mixture (90/10, v/v) of ammonium formate (10 mmol/l, pH 8.2) and methanol. Using the same solvent mixture as the mobile phase and monitoring the effluent at 330 nm, the target compound was eluted between 460 and 740 mL as confirmed by RP-HPLC/taste testing. After freeze-drying, (*S*)-N-(1-carboxy-2-phenylethyl)-6-hydroxymethylpyridinium-3-olate (2.7 mmol, 11 % in yield) was obtained with a purity of more than 99% **(A8).**

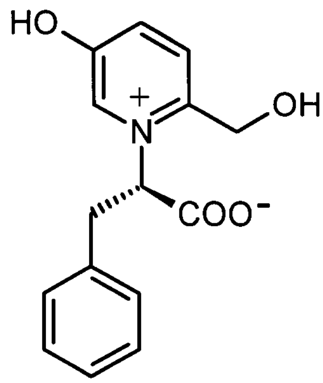

(*S*)-Phepyridaine **(A8)** at neutral pH

**Spectroscopic data of (*S*)-Phepyridaine:**

**LC/MS** (ESI) : 274 (100, $[M+1]^+$), 547 (10, $[2 M+1]^+$).
**UV/VIS** (ammonium formate [10 mmol/l, pH 8.2] / methanol; 85/15; v/v) : $\square_{max}$ 254 nm, 330 nm.
**$^1$H NMR** (400 MHz; MeOH-$D_4$, GRASP-COSY): δ 3.36 - 3.42 (dd, 1H, J=11.0, 14.7 Hz, $CH_2$), 3.80 - 3.85 (dd, 1H, J=4.5, 14.7 Hz, $CH_2$), 4.37 - 4.40 (d, 1H, J=14.8 Hz, $CH_2$), 4.477 - 4.50 (d, 1H, J=14.8 Hz, $CH_2$), 5.55 - 5.59 (dd, 1H, J=4.5, 11.0 Hz, CH), 7.14 - 7.22 (m, 5 x 1H, ArH; 1 x 1H, CH), 7.51 - 7.54 (m, 2 x 1H, CH).
**$^{13}$C NMR** (400 MHz; MeOH-$D_4$; DEPT, HSQC, HMBC): δ 39.4 [$CH_2$], 59.5 [$CH_2$], 71.1 [CH], 127.3 [ArH], 127.5 [CH], 128.9 [2 x ArH], 129.0 [2 x ArH], 131.0 [CH], 132.7 [CH], 136.6 [Ar], 143.6 [C], 161.2 [CO], 170.7 [COOH].

**Syntheses of (S)-N-(1,3-dicarboxypropyl)-6-hydroxymethylpyridinium-3-olate [(*S*)-Glupyridaine] ( R1 is the rest of L-glutamic acid )**

***From 5-(hydroxymethyl)-2-furanaldehyde (HMF) and L-glutamic acid.***

A solution of 5-(hydroxymethyl)-2-furanaldehyde (25 mmol) and monosodium L-glutamatic acid salt (50 mmol) in water/ethanol (1/1, v/v; 40 mL) was adjusted to pH 9.4 with conc. sodium hydroxide solution and then refluxed for 2 days. After cooling to room temperature the reaction mixture was freed from solvent *in vacuo* (45 mbar), taken up in aqueous trifluoroacetic acid (0.1 %; 20 mL), and the aqueous solution was extracted with ethyl acetate (2 × 10 mL). Aliquots (5 mL) of the aqueous phase were then applied onto the top of a water-cooled glass column (4 x 20 cm) filled with a slurry of RP-18 material (LiChroprep 25 - 40 µm, Merck) in aqueous trifluoroacetic acid (0.1 %). Using the same solvent as the mobile phase and monitoring the effluent at 300 nm, an umami-like tasting compound was eluted between 90 and 120 mL as confirmed by RP-HPLC/taste testing. After freeze-drying, these fractions were freeze-dried, the residue was taken up in aqueous trifluoroacetic acid (0.1 %; 5 mL) and (*S*)-N-(1,3-dicarboxypropyl)-6-hydroxymethyl-pyridinium-3-olate (2.3 mmol, 9 % yield, **(A5)**) was isolated by RP-18 HPLC with a mixture (1/99) of methanol and ammonium formate (10 mmol/L; pH 8.2).

**Spectroscopic data of (*S*)-Glupyridaine:**

**LC/MS** (ESI): 256 (65; $[M+1]^+$), 511 [42; $2M+1]^+$), 533 [11; $2M+Na]^+$), 766 [100; $3M+1]^+$), 788 [46; $3M+Na]^+$).
**UV/VIS** (0.1% TFA in water, pH 2.2): $\lambda_{max}$ = 299 nm.
**UV/VIS** (ammonium formate; 10 mmol/L; pH 8.2): $\lambda_{max}$ = 251, 330 nm.
**$^1$H NMR** (360 MHz; DMSO-$d_6$, GRASP-COSY): δ 2.01-2.22 (m, 2H, $CH_2$), 2.46-2.48 (m, 2H, $CH_2$), 4.60 (s, 2H, $CH_2$), 4.96-4.99 (t, J=7.5 Hz, 1H, CH), 7.36-7.38 (d, 1H, J=7.90, CH), 7.55-7.57 (d, J=8.8 Hz, 1H, CH), 7.95 (s, 1H, CH), 8.36 (bs, 2H, 2×COOH).
**$^{13}$C NMR** (360 MHz; DMSO-$d_6$, DEPT, HSQC, HMBC): δ 29.4 [$CH_2$], 32.7 [$CH_2$], 59.6 [$CH_2$], 69.1 [CH], 128.0 [CH], 132.5 [CH], 133.0 [CH], 140.1 [C], 163.3 [C], 165.8 [COOH], 174.9 [COOH].

**Synthesis of N-(Gly-Phe)-6-hydroxymethyl-pyridinium-3-olate [(*S*)-GlyPhepyridaine] ( R1 is the rest of glycine from the dipeptide H-Gly-Phe-OH )**

***1. N-(Gly-Phe)-2-hydroxymethyl-5-(methylamino)furane.***

A solution of 5-(hydroxymethyl)-2-furanaldehyde (5 mmol) and H-Gly-Phe-OH (10 mmol) in water (15 ml) was adjusted to pH 9.4 with conc. sodium hydroxide solution and stirred in a hydrogenation vessel for 30 min at room temperature. After the addition of Raney nickel (0.5g) the solution was stirred in a hydrogen atmosphere (5 bar) for 40h. The catalyst was filtered off, washed with methanol, and the filtrate was concentrated in *vacuo*. The residue was taken up in ammonium formate buffer (10 mmol/l, pH 8.2) and applied onto the top of a water-cooled glass column (4 x 30 cm) filled with a slurry of RP-18 material (LiChroprep 25 - 40 μm, Merck) in a mixture (75/25, v/v) of ammonium formate (10 mmol/l, pH 8.2) and methanol. Using the same solvent mixture as the mobile phase and monitoring the effluent at 220nm, the target compound was eluted between 480 and 800 mL as confirmed by RP-HPLC/taste testing. After freeze-drying, *N*-(Gly-Phe)-2-hydroxymethyl-5-(methylamino)furan (2.6 mmol, 53 % yield) was obtained.

**Spectroscopic data of *N-* (Gly-Phe)-2-hydroxymethyl-5-(methylamino)furan:**

**LC/MS** (ESI): 333 (37, $[M+1]^+$), 355 (68, $[M+Na]^+$), 655 (82, $[2 M+1]^+$), 709 (92, $[2 M+ 2 Na]^+$), 731 (100, $[2 M+ 3 Na]^+$).

**$^1$H NMR** (400 MHz; DMSO-$d_6$) : δ 2.91 - 2.96 (dd, 1H, J=6.7, 13.5 Hz, $CH_aH$), 3.02 - 3.04 (d, 2H, J=4.1 Hz, $CH_2$), 3.07 - 3.12 (dd, 1H, J=12.8, 13.5 Hz, $CH_bH$), 3.49 (s, 2H, $CH_2$), 4.24 - 4.29 (dd, 1H, J=6.7, 12.8 Hz, CH), 4.34 (s, 2H, $CH_2$), 6.08 - 6.09 (d, 1H, J=3.1 Hz, CH), 6.16 - 6.17 (d, 1H, J=3.1 Hz, CH), 7.13 - 7.23 (m, 5H, 5 x ArH).

**$^{13}$C NMR** (400 MHz; DMSO-$d_6$; HMQC, HMBC): δ 37.9 [$CH_2$], 45.8 [$CH_2$], 51.6 [$CH_2$], 55.0 [CH], 57.3 [$CH_2$], 107.8 [CH], 108.8 [CH], 127.1 [ArH], 128.5 [2xArH], 129.8 [2xArH], 136.7 [Ar], 153.3 [C], 154.8 [C], 170.4 [CO], 172.0 [COOH].

***2. N-(Gly-Phe)-6-hydroxymethyl-pyridinium-3-olate [ (S) - GlyPhepyridaine ].***

To a cooled (0°C) solution of N-(Gly-Phe)-2-hydroxymethyl-5-(methylamino)furan (2.5 mmol) in 15 mL of water was added drop-wise a solution of bromine in methanol (2.5 mmol in 3.5 mL) over a period of 30 min and stirring was continued for 1h. Rests of bromine were reduced with sodium thiosulfate (2 mL of a 0.5M solution) and the resulting mixture was neutralized by the addition of a strongly basic ion exchange resin (Ion exchanger III, Merck), filtered and evaporated. The residue was taken up in a mixture (90/10, v/v) of formic acid (0.1% in water) and methanol and applied onto the top of a water-cooled glass column (4 x 30 cm) filled with a slurry of RP-18 material (LiChroprep 25 - 40 μm, Merck) in a mixture (90/10, v/v) of formic acid (0.1% in water) and methanol. Using the same solvent as the mobile phase and monitoring the effluent at 300 nm, the target compound was eluted between 500 and 650 mL as confirmed by RP-HPLC/taste testing. After freeze-drying twice, *N*-(Gly-Phe)-6-hydroxymethyl-pyridinium-3-olate **(A9)** was obtained (0.9 mmol, 36 % yield).

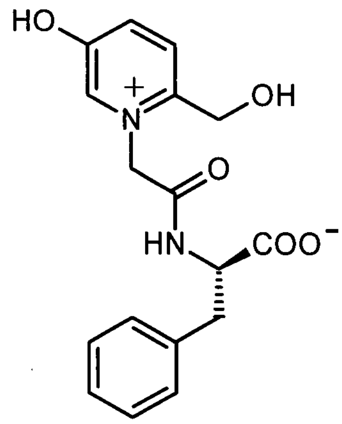

(*S*)-GlyPhepyridaine **(A9)** at neutral pH

**Spectroscopical data of *N*-(Gly-Phe)-6-hydroxymethylpyridinium-3-olate:**

**LC/MS** (ESI): 331 (100, $[M+1]^+$), 661 (25, $[2 M+1]^+$), 683 (33, $[2 M+Na]^+$), 705 (35, $[2 M+ 2Na]^+$), 727 (42, $[2 M+ 3Na]^+$).
**$^1$H NMR** (400 MHz; DMSO-$d_6$): δ 2.81- 2.86 (dd, 1H, J=8.4, 13.5, $CH_aH$), 3.09 - 3.13 (dd, 1H, J=3.9, 13.5 Hz, $CH_bH$), 4.17 - 4.22 (dd, 1H, J=3.9, 8.4 Hz, CH), 4.35 (s, 2H, $CH_2$), 5.13 (s, 2H, $CH_2$), 7.13 - 7.27 (m, 6H, CH, 5xArH), 7.42 - 7.45 (d, 1H, J=9.3 Hz, CH), 7.68 - 7.69 (d, 1H, J=1.8 Hz, CH).
**$^{13}$C NMR** (400 MHz; DMSO-$d_6$; HMQC, HMBC) : δ 37.4 [$CH_2$], 56.5 [$CH_2$], 58.0 [$CH_2$], 58.6 [CH], 126.2 [ArH], 127.5 [CH], 128.3 [2 x ArH], 129.6 [2 x ArH], 133.4 [CH], 136.6 [CH], 137.5 [Ar], 139.3 [C], 165.1 [CO], 166.2 [CO], 173.8 [COOH].

<u>**Sensory analyses**</u>

**Sensory panel training:**

Assessors were trained to evaluate the taste of aqueous solutions (1 mL each) of the following standard taste compounds by using a triangle test: saccharose (50 mmol/L) for sweet taste; lactic acid (20 mmol/L) for sour taste; NaCl (12 mmol/L) for salty taste; caffeine (1 mmol/L) for bitter taste; monosodium glutamate (MSG, 8 mmol/L, pH 5.7) for umami taste; tannin (gallustannic acid; 0.05%) for astringency. Sensory analyses were performed in a sensory panel room at 22-25°C on three different sessions.

**Taste thresholds:**

The taste thresholds of the tastants or mixtures containing basic tastants plus pyridinium betains were determined in a triangle test using tap water as the solvent (H. Wieser and H.-D. Belitz, *Z. Lebensm. Unters. Forsch.* 1975, *159,* 65-72). The samples (3 mL) were presented in order of increasing concentrations (serial 1:1 dilutions). The threshold values evaluated in two different sessions were averaged.
The values between individuals and separate sessions differed not more than one dilution step; i.e., a threshold value of 0.5 mmol/L for caffeine represents a range from 0.25 to 1.0 mmol/L. Prior to sensory analysis, the purity of the synthetic taste compounds was proven by LC/MS and $^1$H NMR spectroscopy.

**Beef taste recombinate (mg/L water):**

Threonine (68), serine (38), MSG (64), proline (16), glycine (56), alanine (192), valine (34), methionine (20), isoleucine (16), leucine (28), tyrosine (38), phenylalanine (26), lysine hydrochloride (44), histidine (38), arginine (32), taurine (248), ribose (4), mannose (8), fructose (20), glucose (40), IMP·2Na·$5H_20$ (424), GMP·2Na·$7H_20$ (80), AMP (60), pyroglutamic acid (1296), lactic acid (3567), succinic acid (98), NaCl (854), KCl (3940), $MgCl_2$·$6H_20$ (1104),

$CaCl_2 \cdot 2H_20$ (14), $H_3PO_4$, carnosine (912), creatine (1106), and creatinine (572) were dissolved in 1L of water, and the pH was adjusted to 5.0 (K. Shima et al., *J. Agric. Food. Chem.* **1998,** 46, 1465-1468).

**Taste profiling:**

The sensory panel was asked to score the given taste qualities of the biomimetic beef taste recombinate on a scale from 0 (not detectable) to 5 (strong). To study the influence of (*S*)-Alapyridaine on the taste qualities of that recombinate, a triangle test was performed with two glass vials containing the pure beef taste recombinate, and one glass vial containing the beef taste recombinate plus a known amount of (*S*)-Alapyridaine. The sensory panel was asked to detect the (*S*)-Alapyridaine-containing solution out of the three vials and to rank the given taste qualities on a scale from 0 to 5 in their intensity.

**Determination of iso-intensity:**

The taste intensities of aqueous solutions containing a basic tastant (glucose, MSG, or L-phenylalanine) in constant concentrations and (*S*)-Alapyridaine in various concentrations were compared with those of a solution containing the basic tastant only. To achieve this, triangle tests were performed with two glass vials containing the basic tastant in constant concentrations, and one glass vial containing a stepwise 1:1-diluted mixture of basic tastant and (*S*)-Alapyridaine. The sensory panel was asked to detect the dilution at which all three glass vials showed the same intensity in taste. The relative taste strength (taste enhancing factor) of a mixture containing the basic tastant and (*S*) -Alapyridaine was related to the basic tastants glucose, MSG, or phenylalanine, respectively, as standard substances, and was determined as the ratio of the concentrations **c** of iso-intensely tasting solutions of the basic tastant, such as glucose (glc), and the mixture containing tastant plus pyridinium betain, i.e.

$$f_{glc,g}(c_{glc}) = c_{glc} / c_{glc} + \text{Alapyridaine}$$

Surprisingly, certain Pyridinium Betains of the general formula **(A)** were found to be taste-active compounds (Table 1). Moreover, they may have significantly lower taste thresholds as compared to the corresponding amino acids. The (*S*)-Glupyridaine was evaluated with the lowest threshold concentration of 0.3-0.45 mmol/kg water, which is by a factor of eight below the threshold determined for MSG (Table 1). The novel pyridinium-3-olate showed the same taste attributes as the corresponding amino acids, but with 8-10 times lower threshold concentrations.

For example, (*S*)-Alapyridaine was evaluated with a threshold concentration of 1.0-1.5 mmol/L, which showed an 80-fold and 10-fold higher taste activity as compared to glucose and L-alanine, respectively (Table 1). The novel (*S*)-Alapyridaine was, however, not only an intense sweetener, but imparts a very pleasant sweet taste quality without any undesirable off-taste such as, e.g., metallic, which is often perceived when using artificial sweeteners.

Table 1.

| Comparison of detection thresholds of glucose, amino acids (= AA) and taste-active Pyridinium Betains (= Pyridaine) | | | |
|---|---|---|---|
| Taste compound | Taste quality | Detection threshold [mmol/kg water, pH 7.0] | Threshold ratio (AA/Pyridaine) |
| Glucose | Sweet | 80.0 | |
| L-alanine | Sweet | 12.0 | |
| (*S*)-**Ala**pyridaine | Sweet | 1.0-1.5 | 10 |
| L-phenylalanine | Bitter | 16.0 | |
| (*S*)-**Phe**pyridaine | Bitter | 1.5-2.5 | 8 |
| MSG | Umami-like | 3.0 | |
| (*S*)-**Glu**pyridaine | Umami-like | 0.3-0.45 in water 0.05-0.07 in 0.5% NaCl | 8 |

The taste enhancing effect of the new compounds of the general formula **(A)** is another surprising findings. As shown in Table 2, an equimolar mixture of L-alanine and (*S*)-Alapyridaine leads to a sweet tasting solution with a threshold value of 0.8-1.2 mmol/kg water, which is by a factor of 12 lower as compared to that of L-alanine.

Similarly, the mixture of L-phenylalanine and (*S*)-Phepyridaine showed a taste threshold by a factor of 11 lower than that of L-phenylalanine (Table 2). That means, the taste of the mixture is more pronounced than that of the individual components although only half of the Pyridinium Betain amount is present in the mixtures.

Table 2.

| Comparison of detection thresholds of L-alanine, (*S*)-Alapyridaine and an equimolar mixture of L-alanine and (*S*)-Alapyridaine as well as L-phenylalanine, (*S*)-Phepyridaine and an equimolar mixture of L-phenylalanine and (*S*)-Phepyridaine. | | | |
|---|---|---|---|
| Taste compound(s) | Taste quality | Detection threshold [mmol/kg water, pH 7.0] | Threshold decrease |
| L-Alanine | Sweet | 12.0 | |
| (*S*)-**Ala**pyridaine | Sweet | 1.0-1.5 | 10 |
| L-Alanine + (*S*)-**Ala**pyridaine (1+1) | Sweet | 0.8-1.2 | 12 |
| L-**Phe**nylalanine | Bitter | 16.0 | |
| (*S*)-Phepyridaine | Bitter | 1.5-2.5 | 8 |
| L-Phenylalanine + (*S*)-**Phe**pyridaine (1+1) | Bitter | 1.0-2.0 | 11 |

The sweet taste of (*S*)-Alapyridaine is by a factor of 64 lower than that of glucose (Table 3). Surprisingly, it was found that (*S*)-Alapyridaine not only enhances the sweet taste of the corresponding amino acid L-alanine, as shown in Table 2, but also that of glucose (Table 3). This taste enhancing effect is evidenced by the taste threshold of an equimolar mixture of glucose and (*S*)-Alapyridaine, i.e. 0.5-1.0 mmol/kg water. This was by a factor of 107 lower as compared to that of glucose, despite the fact that the mixture contained only half of the (*S*)-Alapyridaine amount.

Lowering the amount of (*S*)-Alapyridaine leads to an increase of the taste threshold. The threshold value of the (1+0.25) glucose/(*S*)-Alapyridaine mixture was found to be similar to that (*S*)-Alapyridaine, and by a factor of 64 lower than that of glucose (Table 3). The taste threshold of the (1+0.0313) glucose/(*S*)-Alapyridaine mixture was still 8-fold lower as compared to that of glucose.

Table 3.

| Comparison of detection thresholds of mixtures containing various concentrations of glucose and (*S*)-Alapyridaine. | | | |
|---|---|---|---|
| Taste compound(s) | Taste quality | Detection threshold [mmol/kg water, pH 7.0] | Threshold decrease |
| Glucose | Sweet | 80.0 | |
| (*S*)-**Ala**pyridaine | Sweet | 1.0-1.5 | 64 |
| Glucose + (*S*)-**Ala**pyridaine (**1+1**) | Sweet | 0.5-1.0 | 107 |
| Glucose + (*S*)-**Ala**pyridaine (**1+0.5**) | Sweet | 0.5-1.0 | 107 |
| Glucose + (*S*)-**Ala**pyridaine (**1+0.25**) | Sweet | 1.0-1.5 | 64 |
| Glucose + (*S*)-**Ala**pyridaine (**1+0.125**) | Sweet | 3.0-4.5 | 21 |
| Glucose + (*S*)-**Ala**pyridaine (**1+0.0625**) | Sweet | 5.0-7.5 | 13 |
| Glucose + (*S*)-**Ala**pyridaine (**1+0.0313**) | Sweet | 7.5-11.3 | 8 |

Furthermore, surprisingly the sweet taste of glucose is also enhanced by Pyridinium Betains having other types of taste properties (Table 4). A mixture of equimolar amounts of glucose and the bitter tasting (*S*)-Phepyridaine resulted in a sweet tasting sample showing a threshold value, which was by a factor of 32 lower than that of glucose.

Similarly and even more surprisingly, the tasteless Pyridinium Betain of the dipeptide H-Gly-Phe-OH, i.e. (*S*)-GlyPhepyridaine, showed the same effect as (*S*)-Phepyridaine, indicating the general enhancing potential of Pyridinium Betains. Interestingly, the corresponding dipeptide H-Gly-Phe-OH showed a bitter taste with a threshold value similar to that of L-phenylalanine.

Table 4.

| Comparison of detection thresholds of mixtures of glucose and (*S*)-Alapyridaine, (*S*)-Phepyridaine, or (*S*)-GlyPhepyridaine | | | |
|---|---|---|---|
| Taste compound(s) | Taste quality | Detection threshold [mmol/kg water, pH 7.0] | Threshold decrease |
| Glucose | Sweet | 80.0 | |
| (*S*)-**Ala**pyridaine | Sweet | 1.0-1.5 | 64 |
| Glucose + (*S*)-**Ala-** pyridaine (1+1) | Sweet | 0.5-1.0 | 107 |
| Glucose +(*S*)-**Phe-** pyridaine (1+1) | Sweet | 2.0-3.0 | 32 |
| Glucose + (*S*)-**GlyPhe**pyridaine (1+1) | Sweet | 2.0-3.0 | 32 |

Surprisingly, cetain Pyridinium Betains can also enhance taste properties, which are different from their own taste profile. As shown in Table 5, the sweet tasting (*S*)-Alapyridaine enhances the bitter taste of L-phenylalanine since the threshold value is by a factor of 8 lower than that of the amino acid.

Similarly, (*S*)-Alapyridaine enhances the umami character of MSG by a factor of 5, i.e. the threshold value is by a factor of 5 lower than that of MSG, thus suggesting that the pyridinium betain structure plays a key role in this taste enhancement phenomenon.

Table 5.

| Comparison of detection thresholds of L-phenylalanine, (S)-Phepyridaine, and an equimolar mixture of L-phenylalanine and (*S*)-Alapyridaine as well as L-glutamic acid, (*S*)-Glupyridaine and an equimolar mixture of MSG and (*S*)-Alapyridaine. | | | |
|---|---|---|---|
| Taste compound | Taste quality | Detection threshold [mmol/kg water, pH 7.0] | Threshold decrease |
| L-Phenylalanine | Bitter | 16.0 | |
| (*S*)-**Phe**pyridaine | Bitter | 1.5-2.5 | 8 |
| L-Phenylalanine + (*S*)-**Ala**pyridaine (1+1) | Bitter | 1.5-2.5 | 8 |
| MSG | Umami-like | 3.0 | |
| (*S*)-**Glu**pyridaine | Umami-like | 0.3-0.6 | 7 |
| MSG +(*S*)-**Ala**pyridaine (1+1) | Umami-like | 0.4-0.8 | 5 |

Therefore, the taste enhancing effect of the new compounds of the general formula **(A)** not only applies to the taste of the corresponding amino acid, but also to amino acids having other taste profiles. This cross reactivity phenomenon was also observed with compounds different from amino acids, such as for example glucose.

The taste enhancing potential of the compounds of the general formula **(A)** was further evidenced by evaluating solutions of equal intensity (iso-intensity) containing glucose or mixtures of glucose and, for example, (*S*)-Alapyridaine in various concentrations. As shown in **Figure 1**, the relative sweetness of the mixtures containing glucose and (*S*)-Alapyridaine increases with increasing amount of the Pyridinium Betain compound. This shows the taste enhancing potential of (*S*)-Alapyridaine on the sweet taste of glucose, which is more pronounced at lower glucose concentrations (e.g. 1.5 %).

**Figure 1** shows the relative sweetness $f_{glc}$ (Y axis) of iso-intense solutions of glucose (1.5 or 5.0 % in water) and mixtures containing glucose and (*S*)-Alapyridaine in various concentrations ( X axis represents (*S*)-Alapyridaine in %).

Similar studies with MSG and L-phenylalanine also showed the taste enhancing character of (*S*)-Alapyridaine. For example, an aqueous solution of MSG (0.063 %) and (*S*)-Alapyridaine (0.063 %) developed an umami taste intensity, which when diluted by a factor of 4 resulted in the same umami taste intensity as compared to a solution of MSG (0.063 %). Reducing the amount of (*S*)-Alapyridaine in the mixture to 0.032 % enhanced the umami taste intensity by a factor of 2 as compared to a solution of MSG (0.063 %).

The aqueous solution of L-phenylalanine (0.413 %) and (*S*)-Alapyridaine (0.207 %) developed a bitter taste intensity, which when diluted by a factor of 4 resulted in the same bitter taste intensity as compared to a solution of L-phenylalanine (0.413 %).

**Identification and quantification of (*S*)-Alapyridaine in beef broth**

Beef meat (purchased in a local shop; 1.0 kg), cut into small pieces, was boiled in water (2 L) for 3 h. After cooling to room temperature, the mixture was filtered and the aqueous solution was extracted with ethyl acetate (5 x 200 mL). To remove high-molecular weight compounds, the aqueous phase was separated by ultrafiltration using regenerated cellulose ultrafiltration membranes (Ø 63.5 mm, YM10, Millipore, Bedford, USA) with a cut-off of 1000 Da. The low-molecular weight fraction was freeze-dried, and aliquots (1.0 g) of the non-volatile residue (15.6 g) was fractionated by gel permeation chromatography (GPC) using a water-cooled glass column (400 x 55 mm, Pharmacia) filled with a slurry of Sephadex G-15 (Pharmacia) in aqueous acetic acid (0.1 mol/L). Chromatographic separation was monitored by means of an UV/VIS detector ($\lambda$=254 nm) and the effluent (2.0 ml/min) was collected into 15 mL fractions. These fractions were freeze-dried, the residues were dissolved in water, membrane-filtered, and then analyzed by HPLC (4,6 x 250 mm; BIO-TEK Kontron Instruments, Eching, Germany) using RP-18 material (ODS-Hypersil, 5 $\mu$m, 10 nm, Shandon, Frankfurt, Germany) as the stationary phase. Using a gradient of aqueous trifluoracetic acid (0.1% in water) and methanol as the mobile phase and monitoring the effluent (0.8 mL/min) by means of a diode array detector or a mass spectrometric detector operating in the ESI mode, respectively, (*S*)-Alapyridaine was detected in the GPC fraction eluting between 5.5 and 6.5 h by comparison of the UV/VIS and the LC/MS spectra, the retention times as well as the sensory properties with those obtained for the synthetic reference compound. Quantitative analysis, performed by comparing the peak area obtained at $\lambda$= 298 nm with those of a defined standard solution of the reference compound in methanol, revealed that (*S*)-Alapyridaine is present in beef broth in concentrations of 419 $\mu$g/L, thus verifying the natural occurrence of this taste-active Pyridinium Betain in beef broth and suggesting that in general Pyridinium Betains may occur in thermally treated foods.

**Effect of (S)-Alapyridaine on the taste profile of a "Beef Broth" taste recombinate**

The beef taste recombinate consisting of 16 amino acids, 4 sugars, three 5'-nucleotides, 3 carbonic acids, 3 salts, and 4 other compounds was used as a sample having a basic brothy taste at pH 5 (K. Shima et al., *J. Agric. Food. Chem.* **1998,** 46, 1465-1468). The taste of the reference sample (A) was described as sour, umami, salty and weak sweet and astringent (**Figure 2**). Addition of (*S*)-Alapyridaine (0.5 mg/L) to the recombinate (A) increased the umami character and decreased the sour note in sample (B). Further increase of (*S*)-Alapyridaine (5 mg/L) gave rise to a pronounced umami taste accompanied by a more intense sweet and salty note and a reduced sourness in sample (C) (**Figure 2**).

**Figure 2** shows the Sensory profiling of the taste recombinate (A) and the mixtures with 0.5 mg/L added (*S*)-Alapyridaine (B), and 5.0 mg/L added (S)-Alapyridaine (C).

## Claims

1. Pyridinium-Betain compounds of the following general formula **(A)**

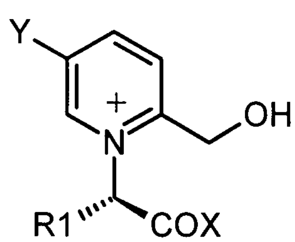

**(A)**

wherein

R1 is taken from the group consisting of a rest of primary L-amino acid,

X is taken from the group consisting of OH and $O^-$
Y is taken from the group consisting of OH and $O^-$

and wherein the counter-ion is taken from the group consisting of sodium, potassium, ammonium, calcium, magnesium, chloride, nitrate, carbonate, sulfate, phosphate, and the like.

**2.** Pyridinium-Betain compounds according to claim 1, wherein R1 is the rest of L-alanine.

**3.** Pyridinium-Betain compounds according to claim 1, wherein R1 is the rest of L-phenylalanine.

**4.** Pyridinium-Betain compounds according to claim 1, wherein R1 is the rest of L-glutamic acid.

**5.** Pyridinium-Betain compounds according to any of claims 1 to 4, wherein

R1 is taken from the group consisting of a rest of primary L-amino acid including glycine,

X of COX is a further amino acid or oligopeptides attached via peptidic bonds comprising primary and secondary L-amino acids including glycine.

**6.** The use of a Pyridinium-Betain compound according to any of claims 1 to 5 as taste active compound, wherein compound **(A)** is added to a food composition for imparting to said food a sweetness, a bitterness, a salty taste or an umami taste.

**7.** The use of a Pyridinium-Betain compound according to any of claims 1 to 5, wherein compound **(A)** is added to a food composition to enhance the sweetness, the bitterness, the salty taste or the umami taste of a compound having said functionality.

**8.** The use according to any of claims 6 or 7, wherein the food composition is taken from the group consisting of chocolate, ice-cream, beverages, sugar confectionery, culinary products, and petfood.

**9.** The use according to any of claims 6 to 8, wherein the amount of the compound **(A)** is comprised between 0.01 and 3000 mg/kg of the whole composition.

**10.** A process for the preparation of the Pyridinium-Betain compounds of claims 1 to 5, wherein compound **(A)** is obtained by synthesis using 5-(hydroxymethyl)-2-' furanaldehyde (HMF) and the corresponding L-amino acids or peptides.

**11.** A process for the preparation of the Pyridinium-Betain compounds of claims 1 to 5, wherein compound **(A)** is obtained by reacting HMF producing precursors, such as mono- and polysaccharides, and degradation products thereof with the corresponding L-amino acids or peptides.

Figure 1

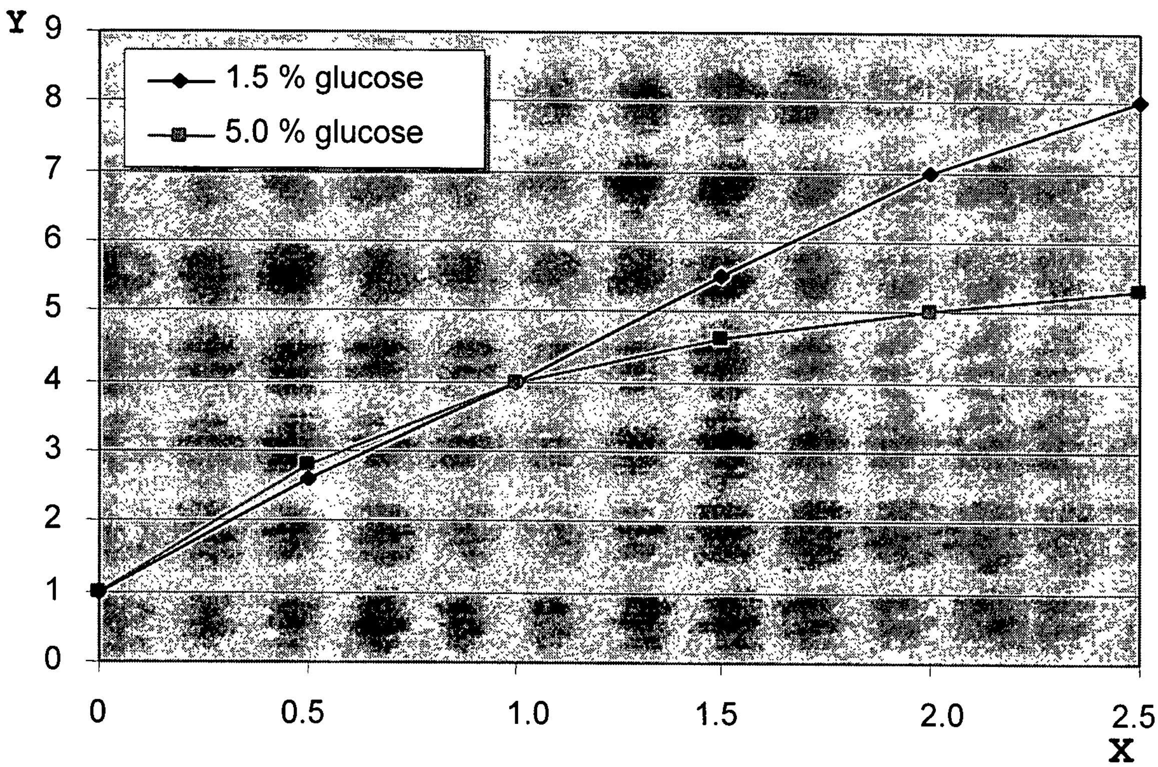

Figure 2

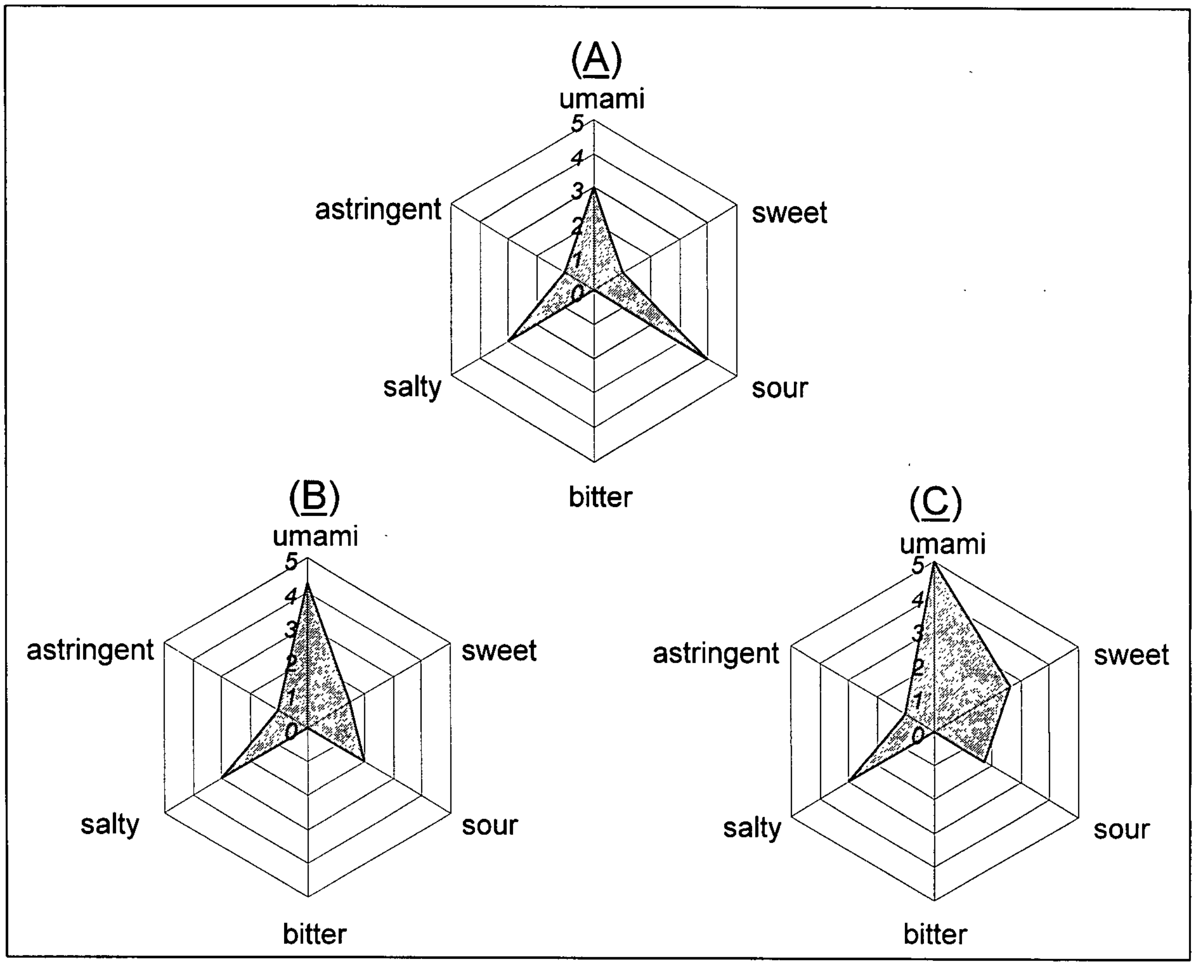
(A)
umami
sweet
sour
bitter
salty
astringent
5
4
3
2
1
0
(B)
umami
sweet
sour
bitter
salty
astringent
5
4
3
2
1
0
(C)
umami
sweet
sour
bitter
salty
astringent
5
4
3
2
1
0

European Patent Office

# EUROPEAN SEARCH REPORT

Application Number
EP 01 12 1349

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 88 06850 A (CENTER INNOVATIV TECHNOLOGY) 22 September 1988 (1988-09-22) * abstract * | 1,5-7 | C07D213/65 A23L1/22 |
| A | US 4 530 799 A (BERTHOLET RAYMOND ET AL) 23 July 1985 (1985-07-23) * claim 1 * | 1,5-7 | |

TECHNICAL FIELDS SEARCHED (Int.Cl.7)
C07D
A23L

The present search report has been drawn up for all claims

| Place of search | Date of completion of the search | Examiner |
|---|---|---|
| THE HAGUE | 17 January 2002 | De Jong, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 12 1349

This annex lists the patent family members relating to the patent documents cited in the above-mentioned European search report. The members are as contained in the European Patent Office EDP file on
The European Patent Office is in no way liable for these particulars which are merely given for the purpose of information.

17-01-2002

| Patent document cited in search report | Publication date | Patent family member(s) | Publication date |
|---|---|---|---|
| WO 8806850 A | 22-09-1988 | AU 1484388 A | 10-10-1988 |
| | | EP 0305469 A1 | 08-03-1989 |
| | | JP 2591812 B2 | 19-03-1997 |
| | | JP 3502517 T | 13-06-1991 |
| | | WO 8806850 A1 | 22-09-1988 |
| | | US 4997672 A | 05-03-1991 |
| US 4530799 A | 23-07-1985 | CH 586653 A5 | 15-04-1977 |
| | | DE 2506151 A1 | 18-09-1975 |
| | | FR 2264004 A1 | 10-10-1975 |
| | | GB 1467535 A | 16-03-1977 |
| | | JP 1220672 C | 26-07-1984 |
| | | JP 50123623 A | 29-09-1975 |
| | | JP 58052986 B | 26-11-1983 |
| | | US 4652711 A | 24-03-1987 |

EPO FORM P0459

For more details about this annex : see Official Journal of the European Patent Office, No. 12/82